(19) Europäisches Patentamt
European Patent Office
Office européen des brevets

(11) Publication number: **0 352 248 A1**

# (12) EUROPEAN PATENT APPLICATION

(21) Application number: 89850234.9

(22) Date of filing: 19.07.89

(51) Int. Cl.[5]: **C 07 H 19/06**
C 07 H 19/073, C 07 H 19/10,
C 07 H 19/16, C 07 H 19/173,
C 07 H 19/20, A 61 K 31/70

(30) Priority: 20.07.88 SE 8802687

(43) Date of publication of application:
24.01.90 Bulletin 90/04

(84) Designated Contracting States:
AT BE CH DE ES FR GB GR IT LI LU NL SE

(71) Applicant: Medivir Aktiebolag
Lundagatan 2 c/o Statens Bakteriologiska Laboratorium
S-105 21 Stockholm (SE)

(72) Inventor: Johansson, Karl Nils Gunnar
Bäverstigen 19
S-150 23 Enhörna (SE)

Lindborg, Björn Gunnar
Helgarögränd 14
S-125 42 Älvsjö (SE)

Noréen, Rolf
Kämpevägen 47
S-151 54 Södertälje (SE)

(74) Representative: Larfeldt, Helene et al
Bergenstråhle & Lindvall AB Sankt Paulsgatan 1
S-116 47 Stockholm (SE)

Claims for the following Contracting States: ES + GR.

## (54) Nucleoside derivatives.

(57) L-ribofuranosyl nucleoside analogues of the formula

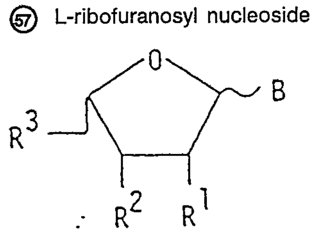

I

wherein B is adenine, guanine, hypoxanthine, 2,6-diaminopurine or

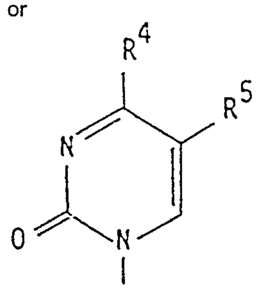

$R^1$ is H, F; $R^3$ is H, OH, F, $N_3$, CN or $R^1$ and $R^2$ together constitute a chemical bond;
$R^3$ is OH or

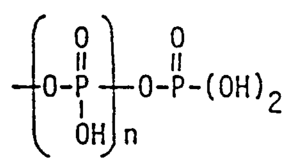

wherein n is 0, 1 or 2; $R^4$ is OH, $NH_2$; $R^5$ is H, $CH_3$ or $C_2H_5$, with certain provisos, in the form of a mixture of alpha and beta anomers or in the form of an alpha or beta anomer for use in therapy in pharmaceutical compositions for therapeutic or prophylactic treatment of infections caused by HIV-viruses, hepatitis B virus or herpes viruses.

EP 0 352 248 A1

**Description**

## Nucleoside derivatives

Field of the invention

The present invention relates to novel chemical compounds and pharmaceutically acceptable salts thereof which can be used in theraphy for therapheutic and prophylactic treatment of the acquired immuno deficiency syndrome (AIDS) and infections caused by viruses requiring reverse transcriptase for replication, such as human immuno deficiency viruses and hepatitis B virus, and also for treatment of other virus diseases, such as those of herpes viruses, diseases which include both common infections and neoplastic diseases, i.e. cancer.

Background of the invention

The effects of viruses on bodily functions is the end result of changes occurring at the cellular and subcellular levels. The pathogenic changes at the cellular level are different for different combinations of viruses and host cells. While some viruses cause a general destruction (killing) of certain cells, other may transform cells into a neoplastic state.

Important common viral infections are herpes dermatitis (including herpes labialis), herpes keratitis, herpes genitalis, herpes zoster, herpes encephalitis, infectious mononucleosis and cytomegalovirus infections all of which are caused by viruses belonging to the herpes virus group. Other important viral diseases are influenza A and B which are caused by influenza A and B virus, respectively. Another important common viral disease is viral hepatitis and especially hepatitis B virus infections are widely spread. Effective and selective antiviral agents are needed for treatment of these diseases as well as for other diseases caused by viruses.

Several different viruses of both DNA and RNA type have been shown to cause tumors in animals. The effect of cancerogenic chemicals can on animals result in activation of latent tumor viruses. It is possible that tumor viruses are involved in human tumors. The most likely human cases known today are leukemias, sarcomas, breast carcinomas, Burkitt lymphomas, nasopharyngeal carcinomas and cervical cancers where RNA tumor viruses and herpes viruses are indicated and papillomas where papilloma viruses are involved. This makes the search for selective inhibitors of tumorogenic viruses and their functions an important undertaking in the efforts to treat cancer.

In the late seventies a new disease was reported, which subsequently was referred to as Acquired Immuno Deficiency Syndrome (AIDS). It is now generally accepted that a retrovirus referred to as HIV (Human Immunodeficiency Virus), formerly known as Human T-cell Lymphotropic Virus (HTLV-III) or Lymphadenopathy Associated Virus (LAV) plays an essential role in the etiology of AIDS. Different types of HIV have been found, such as HIV-1 and HIV-2 and more are likely to be isolated.

AIDS is characterized by a profound immunodeficiency due to low numbers of a subset of lymphocyte-T-helper cells, which are one target for HIV infection. The profound immunodeficiency in AIDS patients makes these patients highly susceptible to a variety of opportunistic infections of bacterial, fungal, protozoal or viral etiology. The etiological agents among viral opportunistic infections are often found in the herpes virus group, i.e. herpes simplex virus (HSV), Varicella Zoster virus (VZV), Epstein-Barr virus (EBV) and, especially, cytomegalovirus (CMV). Other retroviruses affecting humans are HTLV-I and II and examples of retroviruses affecting animals are feline leukemia virus and equine infectious anaemia virus. Human diseases such as multiple sclerosis, psoriasis, tropical spastic paresis and Kawasaki disease have also been reported to be associated with retrovirus infections.

Hepatitis B virus infections cause severe disease such as acute hepatitis, chronic hepatitis, fulminant hepatitis in a considerable number of persons. It is estimated that there are 200 million patients with chronic hepatitis B infection in the world. A considerable number of the chronic cases progress to liver cirrosis and liver tumours. In some cases the hepatitis infections also take a rapid and severe course as in fulminant B hepatitis with about 90 % mortality. At present there is no known effective treatment against hepatitis B infections. The replication of hepatitis B virus is similar to that of retroviruses and it contains the same essential virus reverse transcriptase activity.

Prior art

A great number of nucleoside analogues exhibit several antimetabolic activities. They do so by substituting for or competing with the naturally occuring nucleosides. Recently some nucleoside analogues have been described, which inhibit in cell culture the multiplication of human immunodeficiency virus (HIV, also called HTLV-III, LAV) the causative agent of AIDS and AIDS-related complex (ARC). The naturally occuring nucleosides and most nucleoside analogues described which inhibit HIV multiplication are beta anomers where the sugar ribofuranose has the D-configuration.

The synthesis of the compound β-2′-deoxy-L-uridine has been described by A. Holy in Nucleic Acid Chemistry Vol. 1 (1978) pp 347-353 (Eds L.B. Townsend and R.S. Stuart, Wiley, New York N.Y.) and by A. Holy in Coll. Czech. Chem. Commun. Vol. 37 (1972) pp 4072-4087. In this latter publication the syntheses of β-2′-deoxy-L-thymidine and β-2′-deoxy-L-cytosine also are described.

EP-A2-0 285 884 describes a process to produce α- and β-L-2′,3′-dideoxy-nucleosides and their use as antiviral and antibiotic agents. Said compounds can be represented by the formulas

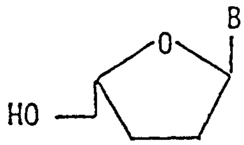

and

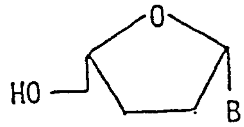

wherein B can be adenine, guanine, hypoxanthine, diaminopurine, uracil, cytosine, thymine and 5-ethyluracil.

Disclosure of the invention

The present invention relates to new L-ribofuranosyl nucleoside analogues of the formula I

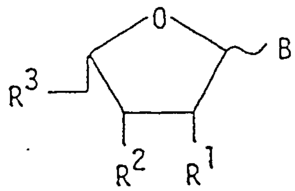

I

wherein B is adenine, guanine, hypoxanthine, 2,6-diaminopurine or

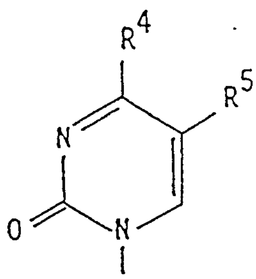

and the radicals $R^1$, $R^2$, $R^3$, $R^4$ and $R^5$ are defined as follows:
$R^1$: H, F;
$R^2$: H, OH, F, $N_3$, CN or $R^1$ and $R^2$ together constitute a chemical bond ;
$R^3$: OH or

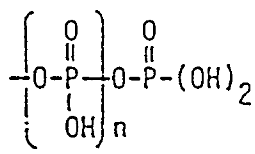

wherein n = 0, 1 or 2 ;
$R^4$: OH, $NH_2$ ;
$R^5$: H, $CH_3$, $C_2H_5$ ;
with the provisos that when $R^1$ is H and $R^3$ is OH, then $R^2$ must not be H, and further that when in the β-anomer $R^1$ is H, $R^2$ is OH and $R^3$ is OH, B is adenine, guanine, hypoxanthine, 2,6-diaminopurine or

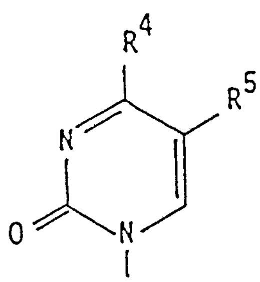

wherein $R^5$ is $C_2H_5$ when $R^4$ is OH and $R^5$ is $CH_3$ or $C_2H_5$ when $R^4$ is $NH_2$; and pharmaceutically acceptable salts thereof.

The compounds of the formula I may have the alpha- or the beta-configuration. According to the Freudenberg convention (1932) the same configuration at the anomeric center (C-1 for aldoses) and the last asymmetric centre (C-4 for pentoses) are termed alpha-anomers. Thus the compounds of the formula I have the configuration Ia (alpha) and Ib (beta)

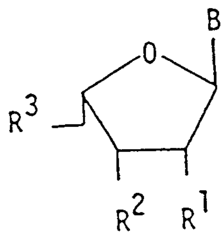

Ia (alpha)

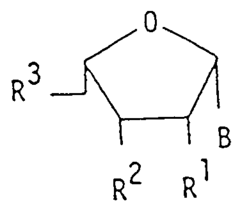

Ib (beta) ;

Said compounds have been found to inhibit the multiplication of human immunodeficiency virus (HIV).

The invention also refers to compounds of the formula I

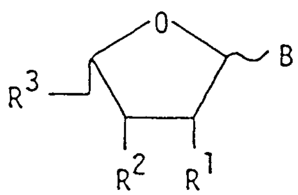

I

wherein B is adenine, guanine, hypoxanthine, 2,6-diaminopurine or

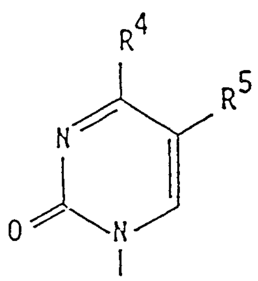

and the radicals $R^1$, $R^2$, $R^3$, $R^4$ and $R^5$ are defined as follows:
$R^1$: H, F;
$R^2$: H, OH, F, $N_3$, CN or $R^1$ and $R^2$ together constitute a chemical bond ;
$R^3$: OH or

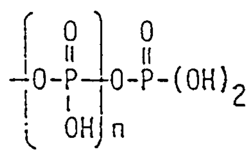

wherein n = 0, 1 or 2 ;
$R^4$: OH, $NH_2$ ;
$R^5$: H, $CH_3$, $C_2H_5$; with the proviso that when $R^1$ is H, $R^2$ is H and $R^3$ is OH B is

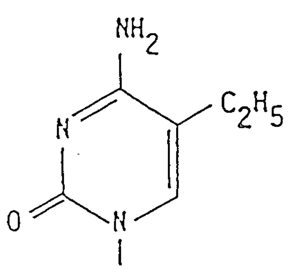

in the form of a mixture of alpha and beta anomers or in the form of an alpha or beta anomer; and pharmaceutically acceptable salts thereof, for use in therapy.

The compounds of the formula I are useful as therapeutic and/or prophylactic agents in the control and treatment of HIV virus infections in man. In a more general aspect, the compounds of the formula I are useful as therapeutic and/or prophylactic agents in the control and treatment of infections caused by retroviruses and hepatitis B virus in mammals and man.

All retroviruses, including HIV, require the enzyme reverse transcriptase in their natural cycle of replication.

Hepatitis B virus (HBV) is a DNA virus with a unique circular double-stranded DNA genome which is partly single-stranded. It contains a specific DNA polymerase required for viral replication. This DNA polymerase also acts as a reverse transcriptase during the replication of HBV DNA via an RNA intermediate.

The compounds of the formula I inhibit the activity of reverse transcriptase of retroviruses including HIV.

A possible area of use for the compounds of the formula I is for treatment of infections caused by hepatitis B virus.

Another possible area of use for the compounds of the formula I is in the treatment of herpes virus infections. Among the herpes viruses may be mentioned Herpes simplex type 1 and 2, varicella (Herpes zoster), virus causing infectious mononucleosis (i.e. Epstein-Barr virus), cytomegalovirus and human herpes virus type 6. Important diseases caused by herpes viruses are herpes dermatitis (including herpes labialis), herpes genitalis, herpes keratitis, herpes encephalitis and herpes zoster.

Another possible area of use for the compounds of the present invention is in the treatment of cancer and tumors, particularly those caused by viruses. This effect may be obtained in different ways, i.e. by inhibiting the transformation of virus-infected cells to a neoplastic state, by inhibiting the spread of viruses from transformed cells to other normal cells and by arresting the growth of virus-transformed cells.

The invention furthermore provides:

A pharmaceutical composition comprising a compound of the formula I as an active ingredient and a pharmaceutically acceptable carrier, including lipsomes; and

A method for therapeutic and/or prophylactic treatment of virus infections in an animal or human host in need of treatment comprising administering an effective amount of a compound of the formula I.

It is a preferred aspect of the invention to treat infections caused by viruses requiring reverse transcriptase for replication, including human immuno deficiency viruses and hepatitis B virus.

The invention also relates to the use of a compound of the formula I for the manufacture of a medicament for therapeutic and/or prophylactic treatment of the acquired immuno deficiency syndrome and infections caused by viruses requiring reverse transcriptase for replication.

Preferably they can be used for the treatment of infections caused by HIV viruses or hepatitis B virus.

Preferred compounds of the formula I

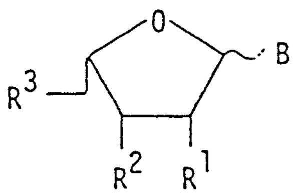

I

are those wherein

$R^1$ is H, F

$R^2$ is H, F, $N_3$

$R^1$ and $R^2$ together constitute a chemical bond

$R^3$ is OH or

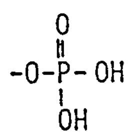

i.e. a monophosphate ester thereof

$R^4$ is OH, $NH_2$

$R^5$ is H, $CH_3$

Examples of especially preferred compounds are those of the formula I wherein

| $R^1$ | $R^2$ | $R^3$ | $R^4$ | $R^5$ |
|---|---|---|---|---|
| H | F | OH | $NH_2$ | H |
| H | F | OH | $NH_2$ | $CH_3$ |
| H | F | OH | OH | $CH_3$ |
| H | $N_3$ | OH | OH | $CH_3$ |
| —— | —— | OH | $NH_2$ | H |
| —— | —— | OH | OH | $CH_3$ |
| H | H | $OPO_3H_2$ | $NH_2$ | H |
| H | H | $OPO_3H_2$ | $NH_2$ | $CH_3$ |
| H | H | $OPO_3H_2$ | OH | $CH_3$ |

Examples of pharmaceutically acceptable salts of the compounds of formula I include base salts, e.g. derived from an appropriate base, such as alkali metal (e.g. sodium, potassium, alkaline earth metal, e.g. magnesium) salts, ammonium and $NX_4^+$ (wherein X is $C_{1-4}$ alkyl). Physiologically acceptable acid salts include salts of organic carboxylic acids such as acetic, lactic, gluconic, citric, tartaric, maleic, malic, pantothenic, isethionic, oxalic, lactobionic and succinic acids; organic sulfonic acids such as methanesulfonic, ethanesulfonic, benzenesulfonic, p-chlorobenzenesulfonic and p-toluenesulfonic acids and inorganic acids such as hydrochloric, hydroiodic, sulfuric, phosphoric and sulfamic acids.

Mono-, di- and triphosphate esters of the compounds are also included in the invention. Physiologically acceptable counterions of the phosphate groups include inorganic and organic counterions. Inorganic counterions are for example ammonium, sodium, potassium, lithium, magnesium and calcium. Organic counterions are derived from non-toxic bases, such as primary, secondary and tertiary amines, including naturally occuring amines. Examples of such amines are diethylamine, triethylamine, isopropylamine; ethanolamine, morpholine, 2-diethylaminoethanol, glucosamine, N-methylglucamine, piperazine and dicyclohexylamine.

In clinical practice the nucleoside analogues of the formula I will normally be administered orally, by injection or by infusion in the form of a pharmaceutical preparation comprising the active ingredient in the form of the original compound or optionally in the form of a pharmaceutically acceptable salt thereof, in association with a pharmaceutically acceptable carrier which may be a solid, semi-solid or liquid diluent or an ingestible capsule. The compound may also be used without carrier material. As examples of pharmaceutical preparations may be mentioned tablets, dragées, capsules, granulates, suspensions, elixirs, syrups, solutions, liposomes etc. Usually the active substance will comprise between 0.05 and 20 % for preparations intended for injection and between 10 and 90 % for preparations intended for oral adminsitration.

In the treatment of patients suffering from retrovirus, especially HIV, or hepatitis B virus infections, it will be preferred to administer the compounds by any suitable route including the oral, parenteral, rectal, nasal, topical and vaginal route. The parenteral route includes subcutaneous, intramuscular, intravenous and sublingual administration. The topical route includes buccal and sublingual administration. The dosage at which the active ingredients are administered may vary within a wide range and will depend on various factors such as the severity of the infection, the age of the patient etc., and may have to be individually adjusted. As a possible range for the amount of the compounds of the invention or a physiologically acceptable salt thereof to be administered per day may be mentioned from about 10 mg to about 10 000 mg, preferentially 100-500 mg for intravenous administration and preferentially 100-3000 mg for oral administration.

Compounds of the formula I can cooperate synergistically or additively with a wide range of other therapeutic agents, thereby enhancing the therapeutic potential of both agents without adding the toxic effects, thus increasing the therapeutic ratio.

Therefore, a compound of formula I or a pharmaceutically acceptable derivative thereof can be used in combination therapy, wherein the two active agents are present in a ratio resulting in an optimal therapeutic ratio. This can be provided either by a synergistic effect against the viral infection and/or by a decrease in toxicity while maintaining a therapeutic effect which is additive or synergistic.

The optimal therapeutic ratio is observed when the two agents are present in a ratio of 500:1 to 1:500, preferably 100:1 to 1:100, particularly 20:1 to 1:20 and especially 10:1 to 1:10.

Said combination may conveniently be administered together, for example, in a unitary pharmaceutical formulation, or separately for example as a combination of tablets and injections administered at the same time or at different times, in order to achieve the required therapeutic effect.

The compounds of the formula I are potentiated by interferons, other antiviral agents such as foscarnet, AZT, fluorothymidine, HIV protease inhibitors, immunomodulators, intereron inducers and growth factors.

Particularly preferred types of interferon are $\alpha$, $\beta$ and $\gamma$ interferon inducers such as "Ampligen" (Hem

Research).

Other combinations suitable for use according to the present invention include those wherein the second agent is, for example, interleukin II, suramin, foscarnet esters, HPA 23, inhibitors of HIV protease such as pepstatin, steroids, medications such as levamisol or thymosin to increase lymphocyte numbers and/or function as appropriate, or GM-CSF and other factors regulating cell functions.

Methods of preparation

The compounds of the invention may be prepared by general methods, constituting a further aspect of the invention.

Condensing a glycoside as comprised in formula I where the hydroxyl groups may be optionally protected to the N-1 position of a purine or pyrimidine derivative, according to known methods described in the literature. Such methods are described for example in "Basic Principles in Nucleic Acid Chemistry", Vol. 1 (Academic Press, 1974, Ed. P.O.P.Ts'o), in "Nucleoside analogues, Chemistry, Biology and Medical Applications" (Pharma Press, 1979, Eds. R.T. Walker, E. De Clercq and F. Eckstein).

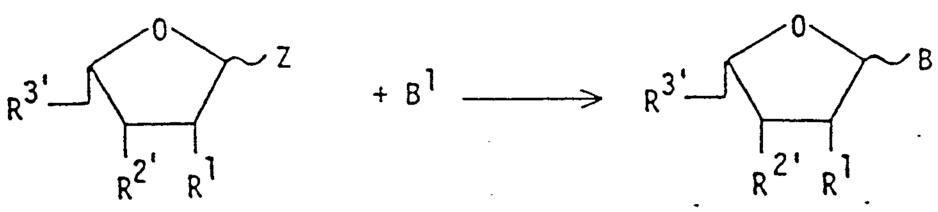

Examples of suitable derivatives of the reacting species are those wherein Z is Cl, Br, I, acyloxy or alkoxy, $R^{2'}$ and $R^{3'}$ is $R^2$ and $R^3$ respectively as defined above with the proviso that when $R^2$ or $R^3$ is OH said OH must be protected as O-acyl, O-benzoyl, O-benzyl or O-silyl (e.g. dimethyl, tert-butylsilyl). The bases $B^1$ may be protected with a silyl protecting group such as $(CH_3)_3Si$ and/or with alkyl och acyl protecting groups. The acyl protecting groups may be used on amino groups of B and alkyl protecting groups (etherderivatives) may be used on carbonyl oxygens of the bases B. After condensation the products may by hydrolyzed or converted by conventional methods, known to those skilled in the art, into compounds of the formula I.

The compounds may also be prepared by a general method whereby the 5′-hydroxymethyl function of a corresponding nucleoside analogue, having the ordidary D-configuration of the ribose moiety, is oxidized to an aldehyde functional group and the stereochemistry at the 4′-position is inverted or racemized, after which the aldehyde group is reduced to a 5′-hydroxymethyl function and the nucleoside analogue having the L-configuration is isolated from the reaction products.

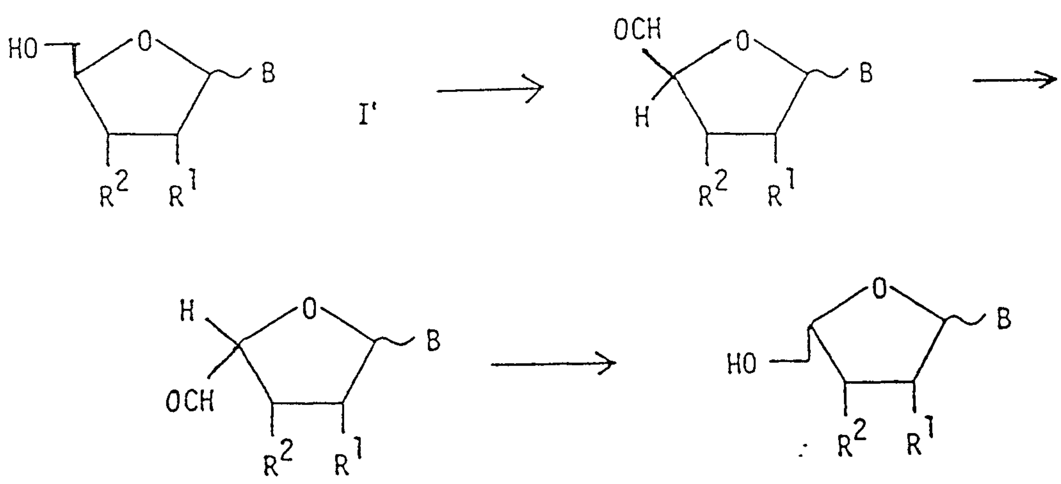


wherein B, $R^1$ and $R^2$ are defined as above.

This reaction sequence has been described for example by J.G. Moffat in Nucleoside Analogues page 88 ff, 1979 (Plenum Press, Eds. R.T. Walker, E. Declercq and F. Eckstein).

Another method for syntheses of various 2 or 3 substituted L-sugar derivatives is to prepare a 2,3-anhydro-L-ribofuranoside or 2,3-anhydro-L-lyxofuranoside analogous to what has been described for the synthesis of 2,3-anhydro-D-ribofuranoside and 2,3-anhydro-D-lyxofuranoside by for example M. Taniguchi et al in Chem. Pharm. Bull. Volume 22, pages 2318 to 2323,, 1974. The 2,3-epoxides would then be reacted with a nucleophile to introduce a fluorine, azido or other functional group and the newly created hydroxylic functional group could be reduced to a hydrogen atom. Finally the so created L-furanoside analogue would be condensed with a purine or pyrimidine base B whereby all the reactions in the sequence are carried out by methods known to those skilled in the arts.

The following examples will further illustrate the invention:

Example 1. 1-(2,3-Dideoxy-alpha,beta-L-ribofuranosyl)-cytosine

1-(2,3-Dideoxy-5-O-tert-butyldiphenylsilyl-alpha, beta-L-ribofuranosyl)-cytosine (170 mg) was dissolved in 2 M sodium hydroxide (5 ml, ethanol-water 1:1) and stirred at ambient temperature for 3 days. Thin layer chromatography (TLC, silica, ethylacetate-methanol 4:1) shows 2 spots, Rf 0.2 and Rf 0.8, for the unprotected title compound and for the free silyl protecting group respectively. Dowex 50Wx8 [pyridinium]$^+$ (2 g) was added, the solution was filtered and the solvent was evaporated in vacuo. The residue was triturated with diethyl ether, dissolved in methanol and filtered through a cotton plug. The solvent was evaporated to give as a residue 1-(2,3-dideoxy-alpha,beta-L-ribofuranosyl)-cytosine (52 mg). The product was a mixture of two anomers in an approximate ratio of 1:6.

$^{13}C$ NMR (Jeol FX 200, DMSO-d6)δ:163,5(d, C-2, α, β); 152.4 (s, C-4); 142.3(d, C-5, α, β); 93.4(d, C-6, α, β); 86.9(d, C-1′, α, β); 82.0(d, C-4′, α, β); 63.0(d, C-5′, α, β); 32.0(d, C-2′, α, β); 25.5(d, C-3′, α,β).

Example 2. 9-(2,3-Dideoxy-alpha,beta-L-ribofuranosyl)-adenine

9-(2,3-Dideoxy-5-O-tert-butyldiphenylsilyl-alpha,beta-L-ribofuranosyl)-adenine (218 mg) was dissolved in 1 M tetrabutylammoniumfluoride in tetrahydrofuran (1 ml) and stirred at ambient temperature for 1 hour. Thin layer chromatography (TLC, silica, ethyl acetate-methanol 5:1) shows 2 spots Rf 0.25 and Rf 0.8, for the unprotected title compound and for the free silyl protecting group respectively. The solvent was evaporated and the crude product was purified by chromatography on a small column of silica (Merck, Kieselgel 60) eluated with ethylacetate-methanol 4:1, followed by purification on a reverse phase plate, RP8, to give 9-(2,3-dideoxy-alpha,beta-L-ribofuranosyl)-adenine (8 mg). The product was a mixture of two anomers in approximately equal amounts.

$^{13}C$ NMR (Jeol FX200, DMSO-d6)δ: 153.9(s,); 141.6(s); 141.3(s); 87.5(d, C-i′, α, β); 83.4(d, C-4′, α, β); 65.0(d, C-5′, α, β); 33.5(d, C-2′, α, β); 25.6(d, C-3′, α, β). Mass spectrum (Jeol DX-300/DA 5000, FAB: 6 kV Xe atoms): 235.1055; $C_{10}H_{14}N_5O_2$ mass 235.1069.

The starting materials for the two compounds in examples 1 and 2 were prepared by the following sequence of reactions a-c:

a) 1-Acetyl-2,3-dideoxy-5-O-tert-butyldiphenylsilyl-alpha, beta-L-ribofuranoside

R-γ-tert-Butyldiphenylsilyloxymethyl-γ-butyrolactone (14 g) in dry diethyl ether (300 ml ) was cooled to -78° C and stirred while diisobutylaluminium hydride in hexane (65 ml, 1.1 M) was added over a period of 30 min. Methanol (15 ml) was added and the reaction solution was slowly warming to room temperature. The solution was extracted with aqueous sodium hydrogencarbonate, dried over magnesium sulfate, and the solvent was evaporated. The residue was dissolved in dry pyridine, acetic anhydride (about 3 equivalents) was added and the reaction solution was heated at 60° C for 4 hours. By TLC (silica, ethylacetate-hexane 1:4) a new spot appears, corresponding to the reaction product, Rf 0.5. The solvent was evaporated in vacuo, the residue was dissolved in diethyl ether, and the new solution was extracted with aqueous sodium hydrogencarborate and dried (sodium sulfate). The solvent was evaporated to give as a residue 1-acetyl-2,3-dideoxy-5-O-tert-butyldiphenylsilyl-alpha,beta-L-ribofuranoside (8.8 g).

$^{13}C$ NMR (Jeol FX 200, $CDCl_3$)δ: 99(d, C-1, α, β); 82(d, C-4, α, β); 66(d, C-5, α, β); 32(d, C-2, α, β); 27(s, $CH_3$, tert-butyl); 25(d, C-3, α, β); 22(s, $CH_3$); 18(s, C, tert-butyl).

b) 1-(2,3-Dideoxy-5-O-tert-butyldiphenylsilyl-alpha,beta-L-ribofuranosyl)-cytosine

Cytosine (0.3 g) in hexamethyldisilazane (4 ml), acetonitrile (4 ml) and chloro trimethylsilane (0.1 ml) under an athmosphere of nitrogen was heated at reflux until a clear solution had formed (about 15 min). The solvent was evaporated in vacuo (1 mm, 40° C) and 1-acetyl-2,3-dideoxy-5-O-tert-butyldiphenylsilyl-alpha,beta-L-ribofuranoside (1.0 g) dissolved in acetonitrile (10 ml) was added. The solution was cooled (0° C) and $SnCl_4$ (0.29 ml) in acetonitrile (5 ml) was added during 1 min. The cooling-bath was removed and the reaction solution was stirred at ambient temperature for 2 hours. By TLL (silica, ethylacetate-methanol, 5:1) a new spot with Rf 0.25 appears, corresponding to the reaction product. Methanol (2 ml) and aqueous ammonia (25 %, 2 ml) were added. The solvent was evaporated to dryness, the residue was dissolved in ethyl acetate, filtered and the solvent was evaporated. Purification by chromatography on silica (Kiselgel Merck 60, 15 % methanol in ethyl acetate) afforded 1-(2,3-dideoxy-5-O-tert-butyldiphenylsilyl-alpha,beta-L-ribofuranosyl)-cytosine (340 mg) as a pure product.

$^{13}C$ NMR (Jeol FX 200, $CD_3OD$)δ: 165.8(s, C-4); 156.2(s, C-2); 140.0(d, C-5, α, β); 94.1(d, C-6, α, β); 87(d, C-1′, α, β); 81.6(d, C-4′, α, β); 65.0(d, C-5′, α, β); 33(d, C-2′, α, β); 26.4(s, $CH_3$, tert-butyl); 25(d, C-3′, α, β); 18.8(s, C, tert-butyl).

c) 9-(2,3-Dideoxy-5-O-tert-butyldiphenylsilyl-alpha,beta-L-ribofuranosyl)-adenine

The title compound was prepared analogous to the synthesis of the corresponding cytosine derivative, 1-(2,3-dideoxy-5-O-tertbutyldiphenylsilyl-alpha,beta-L-ribofuranosyl)-cytosine. The amounts of the various reagents are as follows: adenine (373 mg) heated in hexamethyldisilazane (4 ml), acetonitrile (2.5 ml) and chloro trimethylsilane (0.3 ml). Addition of 1-acetyl-2,3-dideoxy-5-O-tert-butyldiphenylsilyl-alpha,beta-L-ribofuranoside (1.0 g) in acetonitrile (5 ml) cooled to -5° C. $SnCl_4$ (0.29 ml) in acetonitrile (5 ml).

The product 9-(2,3-dideoxy-5-O-tert-butyldiphenylsilyl-alpha,beta-L-ribofuranosyl)-adenine (218 mg) was purified by chromatography on silica (10% methanol in ethyl acetate).
TLC (silica, ethyl acetate-methanol, 9:1) Rf 0.3 $^{13}C$ NMR (Jeol FX 200, $CD_3OD$)δ: 153.1(s, C-2); 141.0(s, C-8); 135-125 (SiPh); 87.2 (d, C-1′, α, β); 83.2(d, C-4′, α, β); 67.0(d, C-5′, α, β); 33.6(d, C-2′, α, β); 27,6(s, CH3, tert-butyl); 26.0(d, C-3′, α, β); 20.3(s, C, tert-butyl).

Example 3. 1-(2,3-Dideoxy-3-fluoro-α-L-ribofuranosyl)-thymine

To a chilled solution of 2′,3′-dideoxy-3′-fluoro-thymidine (108 mg, 0.3 mmol) in $CH_2Cl_2$ (20 ml) at -78°C, DMSO (118 mg, 1.5 mmol) and $(CF_3CO)_2O$ (315 mg, 1.5 mmol) was added. After 15 min, the reaction was warmed up to room temperature and stirred for an additional period of 2 h. Methanol was added to quench the reaction. Volatile matters were removed in vacuo. The residue was redissolved in dry tetrahydrofurane at room temperature and K-tert butoxide (1.1 g, 10 mmol) was added and stirred at 45°C for 2 h under $N_2$. The reaction mixture was then neutralized by addition of 50 % ethanolic-acetic acid. Volatile matters were removed completely by co-evaporation with dry toluene. The residue was redissolved in ethanol (20 ml) and $NaBH_4$ (756 mg, 20 mmol) added and stirred at 50° C to give the title compound in a mixture of different products.

Example 4. 1-(2,3-Dideoxy-3-fluoro-β-L-ribofuranosyl)-thymine

The compound is prepared from 1-(2,3-dideoxy-3-fluoro-α-D-ribofuranosyl)-thymine by a procedure analogous to what has been described in example 3.

This compound can also be prepared starting from 1-(2-deoxy- α -D-threopentofuranosyl)-thymine, which first is oxidized to the 5′-aldehyde and isomerized and then reduced to 1-(2-deoxy-β-L-threo-pentofuranosyl)thymine, analogous to what has been described in Example 3. After protection of the 5′-hydroxyl group, the 3′-hydroxyl group is reacted with diethylamino sulfurtrifluoride (DAST) whereby a fluorine atom is introduced with inversion of configuration and the title compound is formed.

By analogous procedures 1-(2,3-dideoxy-3-azido-β-L-ribofuranosyl)-thymine can also be prepared from 1-(2,3-dideoxy-3-azido-α-D-threopentofuranosyl)thymine, and 1-(2,3-dideoxy-3-fluoro/azido-α-L-ribofuranosyl)thymine can be prepared from 1-(2,3-dideoxy-3-fluoro/azido-β-D-threo-pentofuranosyl)thymine.

Analogously 1-(2,3-dideoxy-3-azido-α-L-ribofuranosyl)-thymine and 1-(2,3-dideoxy-3-azido-β-L-ribofuranosyl)-thymine can also be prepared from their corresponding α- and β-D-ribofuranosyl thymine derivatives respectively.

Biological tests

Test I Effect of compounds of the formula I on HIV in H9 cells

Materials and methods: HIV infection of H9 cells

H9 cells, $10^5$ cells per well on a 24 well plate, suspended in 2 ml RPMI-medium containing 10 % fetal calf serum, 100 µg/ml penicillin, 10 µg/ml streptomycin sulfate and 2 µg/ml polybrene are exposed to HIV (HTLV-$III_B$) and different concentrations of the test compounds. The plates are incubated at 37° C in 5 % $CO_2$ for 6-7 days. The contents in each well is then homogenized with a pipette and transferred to a centrifuge tube. After centrifugation for 10 min at 1500 rpm the supernatant is removed and the cell pellet is analyzed by fixing in methanol on glass plates. Human HIV positive serum diluted 1:80 or 1:160 is added and incubated for 30 min at 37° C. The plate is then washed with phosphate-buffered saline (PBS) containing $Ca^{2+}$ and $Mg^{2+}$. Sheep anti-human conjugate (FITC) is added and after a new incubation the plate is again washed with PBS. Contrast straining is done with Evans blue and after drying the frequency of HIV antigen containing cells is determined in a microscope. The test result is shown in Table 1.

Table 1.

Concentration (µM) for 50 % inhibition ($IC_{50}$) of human immuno deficiency virus multiplication in cell culture

| Compound (code) | $IC_{50}$ µM |
|---|---|
| 1-(2,3-Dideoxy-alpha,beta-L-ribofuranosyl)-cytosine (VSB 815) | < 1 |

Table 1 shows that the tested compound is an active inhibitor of HIV virus multiplication.

Test II Cellular toxicity

H9 cells, $2x10^7$ cells per plate, are incubated in RPMI-1640 medium containing 10 % fetal calf serum, 70 mg/l penicillin, 100 mg/l streptomycin and 10 mM hepes, in absence or presence of test compounds. The number of cells per plate is determined after 48 h. Cells incubated in the absence of test compounds then underwent two

cell division cycles.

F5000 cells, which are human embryo cells, 1x10[5] cells per plate, are incubated in Eagle's minimal essential medium, supplemented with Earle's salts, non-essential amino acids, 10 % fetal calf serum, 10 mM hepes, 70 mg/l penicillin and 100 mg/l streptomycin, in absence or presence of test compounds. The number of cells per plate is determined after 48 h. Cells incubated in the absence of test compounds underwent one cell division cycle. The results are given as % inhibition of cell multiplication when the concentration of the compound is 100 μM or 250 μM. The test results are given in table 2.

Table 2.

Cellular toxity on H9 and F5000 cells

| Compound (code) | % Inhibition H9 | (Conc. μM) F5000 |
|---|---|---|
| 1-(2,3-Dideoxy-alpha,beta-L-ribofuranosyl)-cytosine (VSA 815) | 35 (200) | 55 (100) |

Table 2 shows that the concentrations at which the compound exhibit toxicity exceed the concentration needed to 50 % inhibition of HIV multiplication as given in table 1.

## Claims

1. A compound of the formula

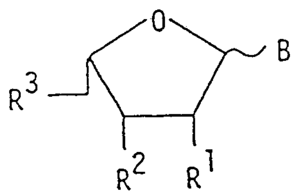

I

wherein B is adenine, guanine, hypoxanthine, 2,6-diaminopurine or

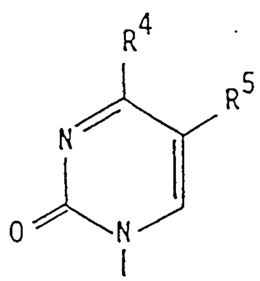

and the radicals $R^1$, $R^2$, $R^3$, $R^4$ and $R^5$ are defined as follows:
$R^1$: H, F;
$R^2$: H, OH, F, $N_3$, CN or $R^1$ and $R^2$ together constitute a chemical bond ;
$R^3$: OH or

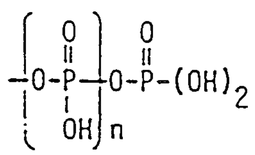

wherein n = 0, 1 or 2 ;
$R^4$: OH, $NH_2$ ;
$R^5$: H, $CH_3$, $C_2H_5$ ;
with the provisos that when $R^1$ is H and $R^3$ is OH, then $R^2$ must not be H, and further that when in the β-anomer $R^1$ is H, $R^2$ is OH and $R^3$ is OH, B is adenine, guanine, hypoxanthine, 2,6-diaminopurine or

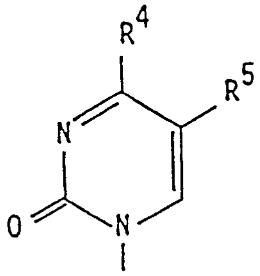

wherein $R^5$ is $C_2H_5$ when $R^4$ is OH and $R^5$ is $CH_3$ or $C_2H_5$ when $R^4$ is $NH_2$; in the form of a mixture of alpha and beta anomers or in the form of an alpha or beta anomer; and pharmaceutically acceptable salts thereof.

2. A compound according to claim 1, wherein $R^3$ is OH.

3. A compound according to any of claims 1-2, wherein $R^1$ and $R^2$ together constitute a chemical bond.

4. A compound according to any of claims 1-2, wherein $R^1$ is H and $R^2$ is F or $N_3$.

5. A compound according to any of claims 1-4, wherein $R^4$ is $NH_2$ and $R^5$ is H or $CH_3$.

6. A compound according to any of claims 1-4, wherein $R^4$ is OH and $R^5$ is H or $CH_3$.

7. A compound of the formula

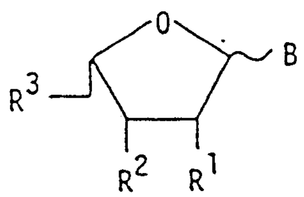

I

wherein B is adenine, guanine, hypoxanthine, 2,6-diaminopurine or

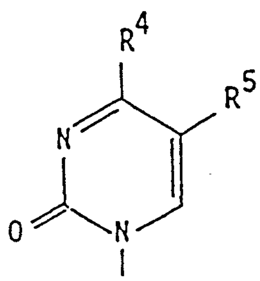

and the radicals $R^1$, $R^2$, $R^3$, $R^4$ and $R^5$ are defined as follows:

$R^1$: H, F;

$R^2$: H, OH, F, $N_3$, CN or $R^1$ and $R^2$ together constitute a chemical bond ;

$R^3$: OH or

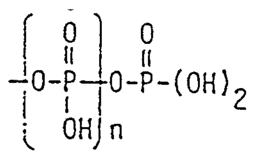

wherein n = 0, 1 or 2 ;

$R^4$: OH, $NH_2$ ;

$R^5$: H, $CH_3$, $C_2H_5$; with the proviso that when $R^1$ is H, $R^2$ is H and $R^3$ is OH B is

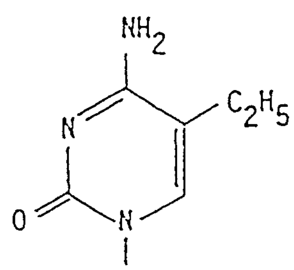

in the form of a mixture of alpha and beta anomers or in the form of an alpha or beta anomer; and pharmaceutically acceptable salts thereof, for use in therapy.

8. A compound of the formula I according to any of claims 1-6 for use in therapy.

9. A pharmaceutical composition comprising as an active ingredient a compound of the formula I according to any of claims 1-7 and a pharmaceutically acceptable carrier, including liposomes.

10. A method for therapeutic and/or prophylactic treatment of virus infections in an animal or human host in need of treatment, comprising administering an effective amount of a compound of the formula I as defined in any of claims 1-7.

11. A method according to claim 10 for treatment of infections caused by viruses requiring reverse transcriptase for replication, including human immuno deficiency virus and hepatitis B virus.

12. A method according to claim 10 for treatment of infections caused by herpes viruses.

13. Use of a compound of the formula I according to any of claims 1-7 for the manufacture of a medicament for therapeutic and/or prophylactic treatment of the acquired immuno deficiency syndrome and infections caused by viruses requiring reverse transcriptase for replication.

14. Use according to claim 13 for the treatment of infections caused by HIV-viruses, hepatitis B virus, or herpes viruses.

15. A process for preparation of a compound of the formula

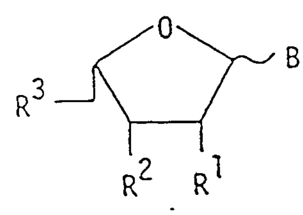

I

wherein B, $R^1$, $R^2$ and $R^3$ are as defined in claim 1, by

a) condensing a glycoside as comprised in the formula I to the N-1 position of a pyrimidine derivative or to the N-9 position of a purine derivative

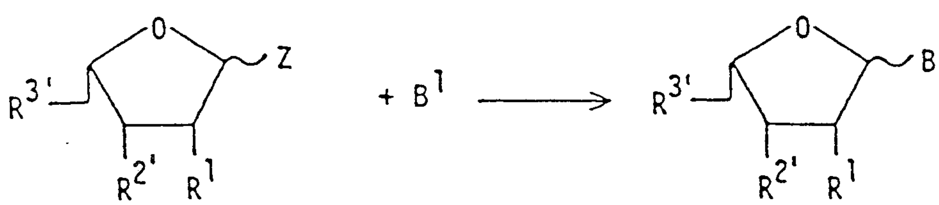

wherein Z is Cl, Br, J, acyloxy or alkoxy, $R^{2'}$ and $R^{3'}$ are $R^2$ and $R^3$, respectively, as defined above or with the proviso that when $R^2$ or $R^3$ is OH then O must have a protecting group, $B^1$ is B as defined above having a silyl, acyl or alkyl protecting group; or

b) oxidizing the 5′-hydroxymethyl function of a D-ribofuranosyl nucleoside analogue of the formula I′ to an aldehyde

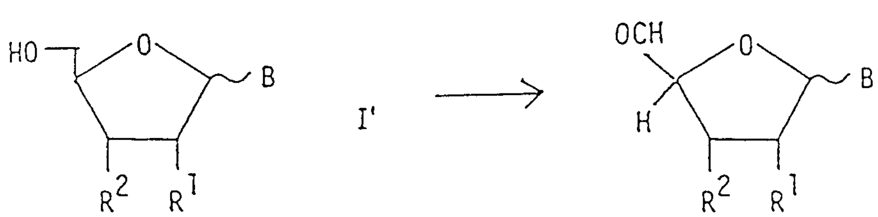

I'

wherein B, $R^1$ and $R^2$ are as defined above, inverting the configuration at the 4′-position, isolating the L-stereomer and reducing it to the L-ribofuranosyl nucleoside analogue of the formula I.

**Claims for the following Contracting States: GR,ES**

1. A process for preparation of a compound of the formula

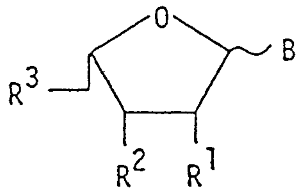

I

wherein B is adenine, guanine, hypoxanthine, 2,6-diaminopurine or

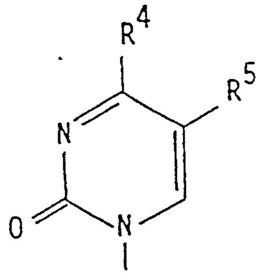

and the radicals $R^1$, $R^2$, $R^3$, $R^4$ and $R^5$ are defined as follows:
$R^1$: H, F;
$R^2$: H, OH, F, $N_3$, CN or $R^1$ and $R^2$ together constitute a chemical bond ;
$R^3$: OH or

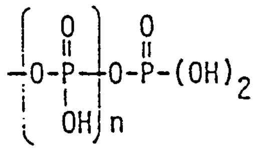

wherein n = 0, 1 or 2 ;
$R^4$: OH, $NH_2$ ;
$R^5$: H, $CH_3$, $C_2H_5$ ;
with the provisos that when $R^1$ is H and $R^3$ is OH, then $R^2$ must not be H, and further that when in the β-anomer $R^1$ is H, $R^2$ is OH and $R^3$ is OH, B is adenine, guanine, hypoxanthine, 2,6-diaminopurine or

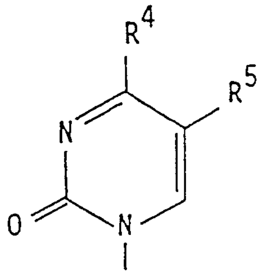

wherein $R^5$ is $C_2H_5$ when $R^4$ is OH and $R^5$ is $CH_3$ or $C_2H_5$ when $R^4$ is $NH_2$, by

a) condensing a glycoside as comprised in the formula I to the N-1 position of a pyrimidine derivative or to the N-9 position of a purine derivative

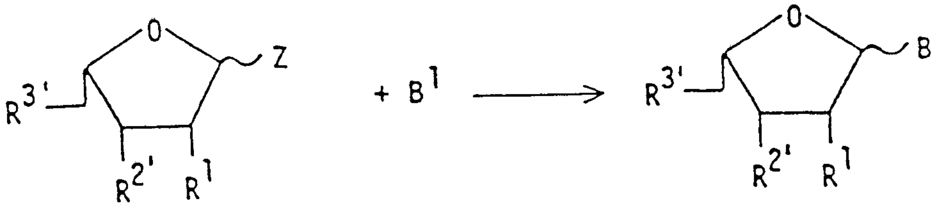

wherein Z is Cl, Br, J, acyloxy or alkoxy, $R^{2'}$ and $R^{3'}$ are $R^2$ and $R^3$, respectively, as defined above or with the proviso that when $R^2$ or $R^3$ is OH then O must have a protecting group, $B^1$ is B as defined above having a silyl, acyl or alkyl protecting group; or

b) oxidizing the 5′-hydroxymethyl function of a D-ribofuranosyl nucleoside analogue of the formula I′ to an aldehyde

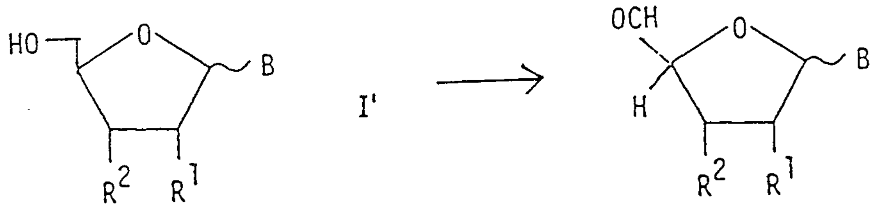

I'

wherein B, $R^1$ and $R^2$ are as defined above, inverting the configuration at the 4′-position, isolating the L-stereomer and reducing it to the L-ribofuranosyl nucleoside analogue of the formula I.

2. A process according to claim 1, wherein $R^3$ is OH.

3. A process according to any of claims 1-2, wherein $R^1$ and $R^2$ together constitute a chemical bond.

4. A process according to any of claims 1-2, wherein $R^1$ is H and $R^2$ is F or $N_3$.

5. A process according to any of claims 1-4, wherein $R^4$ is $NH_2$ and $R^5$ is H or $CH_3$.

6. A process according to any of claims 1-4, wherein $R^4$ is OH and $R^5$ is H or $CH_3$.

7. A process according to any of claims 1-6, wherein the compound of the formula I is obtained in the form of a mixture of alpha and beta anomers or in the form of an alpha or beta anomer and optionally is converted into a pharmaceutically acceptable salt.

8. Use of a compound of the formula

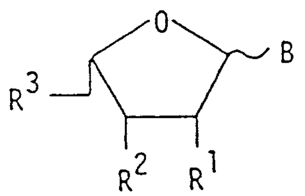

I

wherein B is adenine, guanine, hypoxanthine, 2,6-diaminopurine or

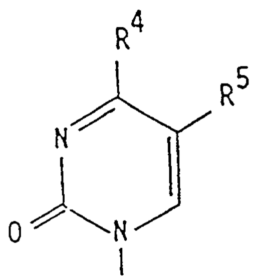

and the radicals $R^1$, $R^2$, $R^3$, $R^4$ and $R^5$ are defined as follows:
$R^1$: H, F;
$R^2$: H, OH, F, $N_3$, CN or $R^1$ and $R^2$ together constitute a chemical bond ;
$R^3$: OH or

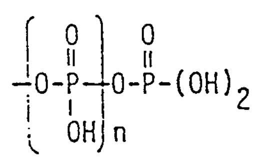

wherein n = 0, 1 or 2 ;
$R^4$: OH, $NH_2$ ;
$R^5$: H, $CH_3$, $C_2H_5$; with the proviso that when $R^1$ is H, $R^2$ is H and $R^3$ is OH B is

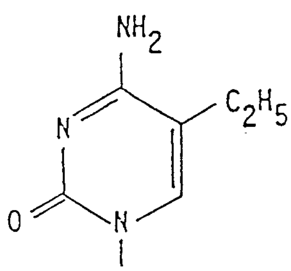

in the form of a mixture of alpha and beta anomers or in the form of an alpha or beta anomer; and pharmaceutically acceptable salts thereof, for the manufacture of a medicament for therapeutic and/or prophylactic treatment of the acquired immuno deficiency syndrome and infections caused by viruses requiring transcriptase for replication.

European Patent Office

**PARTIAL EUROPEAN SEARCH REPORT**
which under Rule 45 of the European Patent Convention shall be considered, for the purposes of subsequent proceedings, as the European search report

Application number
EP 89850234.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,D | EP, A2, 0 285 884 (BRISTOL-MYERS COMPANY) 12 October 1988 * The whole document * --- | 1-9, 13-15 | C 07 H 19/06<br>C 07 H 19/073<br>C 07 H 19/10<br>C 07 H 19/16<br>C 07 H 19/173<br>C 07 H 19/20<br>A 61 K 31/70 |
| X | GB, A, 1 378 408 (CESKOSLOVENSKA AKADEMIE VED) 27 December 1974 * Page 1 lines 10-14, claims * --- | 1-9, 13-15 | |
| X | US, A, 3 817 982 (JULIAN P.H. VERHEYDEN ET AL.) 18 June 1974 * Claims, column 12 line 63 - column 13 line 28 * --- | 1-9, 13-15 | |
| X | EP, A2, 0 261 595 (YALE UNIVERSITY) 30 March 1988 * Claims, page 2 lines 36-47, page 3 lines 39-43, examples 1-3 * --- .../... | 1-9, 13-15 | TECHNICAL FIELDS SEARCHED (Int. Cl.4)<br>C 07 H |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely:

Claims not searched: 10-12

Reason for the limitation of the search:

Method for treatment of the human or animal body by therapy (Art. 52(4) EPC).

| Place of search | Date of completion of the search | Examiner |
|---|---|---|
| STOCKHOLM | 09-10-1989 | CLAESSON G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application number
EP 89850234.9

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| X | EP, A2, 0 199 451 (THE WELLCOME FOUNDATION LIMITED) 29 October 1986 * Claims, column 5 lines 5-34 * --- | 1-9, 13-15 | C 07 H 19/06<br>C 07 H 19/073<br>C 07 H 19/10<br>C 07 H 19/16<br>C 07 H 19/173<br>C 07 H 19/20<br>A 61 K 31/70 |
| X | PROGRESS IN ANTIMICROBIAL AND ANTI-CANCER CHEMOTHERAPY, Proceedings of the 6th International Congress of Chemotherapy, Vol. II, University Park Press, England, 1970, pages 394--397, P LANGEN et al: "5'-deoxy-5'--fluorothymidine and 3'-deoxy-3'-fluoro--thymidine- two thymidine analogues with different points of attack at the molecular level", * The whole article * --- | 1-9, 13-14 | TECHNICAL FIELDS SEARCHED (Int. Cl.4) |
| X | WO, A1, 88/00050 (ASTRA LÄKEMEDEL AKTIEBOLAG) 14 January 1988 * Claims 7-10, page 2 line 20 - page 5 line 3, Experimental tests * --- | 1-9, 13-14 | C 07 H |
| X | EP, A2, 0 206 497 (THE WELLCOME FOUNDATION LIMITED) 30 December 1986 * Claims, page 3 line 1 - line 10 from the bottom, example 12 * --- | 1-9, 13-15 | |
| X | CHEMICAL ABSTRACTS, Vol. 102, No. 5, 4th February 1985, page 19, no. 39565n, Columbus, Ohio, US; WAQAR M. ANWAR et al: "Effects of 2',3'-dideoxynuc-leosides on mammalian cells and viruses", & J. Cell, Physiol. 1984, 121(2), 402-8 (Eng). * Abstract * --- | 1-9, 13-14 | |
| X | DE, A, 1 620 185 (ROBUGEN GMBH) 12 February 1970 * Claim, page 1 last paragraph - page 2 line 18 * --- | 1-9, 13-15 | |
| | -/- | | |

European Patent Office

# PARTIAL EUROPEAN SEARCH REPORT

Application number EP 89850234.9

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| X | WO, A1, 88/04662 (ASTRA LÄKEMEDEL AKTIEBOLAG) 30 June 1988 * Claims 1-5, page 2 line 14 - page 3 line 21, experimental test * --- | 1-9, 13-14 | C 07 H 19/06 C 07 H 19/073 C 07 H 19/10 C 07 H 19/16 C 07 H 19/173 C 07 H 19/20 A 61 K 31/70 |
| X | US, A, 3 457 255 (GORDON H. JONES ET AL.) 22 July 1969 * Claims, abstract, column 1 line 34 - column 2 line 41 * --- | 1-9, 13-15 | |
| X | US, A, 4 659 698 (JEAN-LOUIS IMBACH ET AL.) 21 April 1987 * Column 1 lines 10-28, column 4 line 66 - column 6 line 7 * --- | 1-9, 13-15 | TECHNICAL FIELDS SEARCHED (Int. Cl.4) C 07 H |
| A,D | CHEMICAL ABSTRACTS, Vol. 90, No. 3, 15th January 1979, page 705, no. 23556h, Columbus, Ohio, US; HOLY ANTONIN: "2'-Deoxy-L-uridine: Total synthesis of a uracil 2'-deoxynucleoside from a sugar 2-aminooxazoline through a 2,2'-anhydronucleoside intermediate", & Nucleic Acid Chem. 1978, 1, 347-53 (Eng). * Abstract * --- | 1 | |
| A,D | CHEMICAL ABSTRACTS, Vol. 78, No. 13, 2nd April 1973, page 467, no. 84716j, Columbus, Ohio, US; HOLY ANTONIN: "Nucleic acid components and their analogs. CLIII. Preparation of 2'-deoxy-L-ribonucleosides of the pyrimidine series", & Collect. Czech. Chem. Commun. 1972, 37(12), 4072-87 (Eng). * Abstract * --- | 1 | |